# EUROPÄISCHE PATENTSCHRIFT

(11) **EP 3 241 566 B1**
(45) Veröffentlichungstag und Bekanntmachung des Hinweises auf die Patenterteilung: **07.08.2019**
(21) Anmeldenummer: 17167869.1
(22) Anmeldetag: 25.04.2017
(51) Int. Cl.: A61L 2/07, C02F 5/10

(54) **VERFAHREN UND VORRICHTUNG ZUM ENTKALKEN EINES KÜHLKREISLAUFS FÜR EINE VAKUUMPUMPE FÜR EINEN STERILISATOR**
METHOD AND DEVICE FOR REMOVING INCRUSTATIONS FROM A COOLING CIRCUIT FOR A VACUUM PUMP FOR A STERILISER
PROCÉDÉ ET DISPOSITIF DE DÉTARTRAGE D'UN CIRCUIT DE REFROIDISSEMENT POUR UNE POMPE À VIDE POUR UN STÉRILISATEUR

(30) Priorität: 04.05.2016 DE 102016108268
(43) Veröffentlichungstag der Anmeldung: 08.11.2017
(73) Patentinhaber: Miele & Cie. KG, 33332 Gütersloh (DE)
(72) Erfinder: Karnouschek, Oliver, 5421 Adnet (AT); Gregor, Olaf, 83373 Taching (DE)

(56) Entgegenhaltungen:
- EP-A1- 2 039 750
- WO-A1-02/096472
- CN-U- 203 474 578
- GB-A- 1 459 390
- N:N:: "CHOICE OF DESCALING CHEMICAL AND QUANTITY REQUIRED", , 13. September 2015 (2015-09-13), XP055420025, Gefunden im Internet: URL:http://www.kamco.co.uk/GuidanceNotes/P DF/DescalingAirCompressorCoolers.pdf [gefunden am 2017-10-30]

## Beschreibung

Die Erfindung betrifft ein Verfahren zum Entkalken eines betriebsbereiten Kühlkreislaufs für eine betriebsbereite Vakuumpumpe für einen betriebsbereiten Sterilisator und einen Sterilisator.

Bei einem Sterilisator wird eine Vakuumpumpe des Sterilisators flüssigkeitsgekühlt. Aufgrund der starken Erhitzung der Flüssigkeit im Kühlkreislauf der Vakuumpumpe kann Kalk aus der Flüssigkeit ausfallen und sich im Kühlkreislauf ablagern.

WO 02/096472 offenbart einen Autoklaven mit Vakuumpumpe, welche durch einen Kühlkreislauf gekühlt wird.

Die Firma Kamco vertreibt sogenannte "descaling pumps", mit denen Kühlleitungen enthärtet werden können (URL:http://www.kamco.co.uk).

Die CN 203474578 U beschreibt ein Entkalkungsverfahren für den Wassserkreislauf einer Wasserringpumpe, welche zur Gasabsaugung beim Kohleabbau eingesetzt wird.

Die GB 1459390 A offenbart ein korrosionsminderndes Zugabemittel für Industriewasser.

Die EP2039750 A1 offenbart eine kalklösend wirkende Substanz, welches Zitronensäure enthält.

Der Erfindung stellt sich die Aufgabe, ein verbessertes Verfahren zum Entkalken eines Kühlkreislaufs für eine Vakuumpumpe für einen Sterilisator sowie einen verbesserten Sterilisator bereitzustellen.

Erfindungsgemäß wird diese Aufgabe durch ein Verfahren und einen Sterilisator mit den Merkmalen der unabhängigen Ansprüche 1 und 9 gelöst. Vorteilhafte Ausgestaltungen und Weiterbildungen der Erfindung ergeben sich aus den nachfolgenden Unteransprüchen.

Flüssigkeit in einem Kühlkreislauf kann mit einer kalklösenden Substanz versetzt werden. Durch die Substanz werden das Ausfallen und das Ablagern von Kalk verhindert. Dadurch können Wartungsintervalle und eine Lebensdauer der Vakuumpumpe vergrößert werden.

Es wird ein Verfahren zum Entkalken eines betriebsbereiten Kühlkreislaufs für eine betriebsbereite Vakuumpumpe für einen betriebsbereiten Sterilisator vorgestellt, wobei das Verfahren den folgenden Schritt umfasst:
Fördern zumindest einer kalklösend wirkenden Substanz aus einem Vorratsbehälter in den Kühlkreislauf.

Unter einer kalklösenden Substanz kann ein säurehaltiges Mittel verstanden werden. Beispielsweise kann die Substanz Zitronensäure beinhalten. Die Substanz kann flüssig oder fest vorliegen. Flüssige Substanz kann über eine Pumpe oder unter Ausnutzung der Schwerkraft gefördert werden. Eine feste Substanz kann beispielsweise unter Verwendung einer Zellradschleuse oder Schnecke gefördert werden. Die feste Substanz kann pulverförmig sein oder in Tablettenform vorliegen. Ein Sterilisator kann ein Gerät zum Sterilisieren von beispielsweise medizinisch verwendeten Gegenständen und/oder Materialien sein. Ein Vorratsbehälter kann ein Tank sein. Ein Kühlkreislauf kann beispielsweise die Vakuumpumpe des Sterilisators kühlen. Der Kühlkreislauf kann der Pumpe Flüssigkeit mit niedriger Temperatur zuführen, die in der Pumpe erwärmt wird und als Flüssigkeit mit erhöhter Temperatur wieder über den Kühlkreislauf aus der Pumpe abgeleitet wird. Indem die kalklösend wirkende Substanz dem betriebsbereiten System zugeführt werden kann, ist es nicht erforderlich, den Sterilisator für die Entkalkung außer Betrieb zu setzen oder die Vakuumpumpe oder Leitungen des Kühlkreislaufs zu demontieren.

Die Substanz kann unter Verwendung eines vorgegebenen Zeitplans gefördert werden. Bei einem bekannten Verbrauch der Substanz kann die Substanz kontinuierlich oder diskontinuierlich während des Betriebs des Sterilisators gefördert werden. Dadurch ergibt sich eine einfache und kostengünstige Ansteuerung einer Fördereinrichtung.

Die Substanz kann abhängig von einem, eine Konzentration der Substanz in dem Kühlkreislauf repräsentierenden Konzentrationswert gefördert werden. Die Substanz kann abhängig von einem tatsächlichen Verbrauch der Substanz im Kühlkreislauf gefördert werden. Dadurch kann die Konzentration der Substanz im Kühlwasser innerhalb eines Toleranzbereichs gehalten werden. Eine gute Wirksamkeit der Substanz kann erreicht werden. Der Konzentrationswert kann von einer Erfassungseinrichtung bereitgestellt werden.

Das Verfahren kann einen Schritt des Ermittelns einer zu dosierenden Dosis der Substanz aufweisen. Die Dosis kann abhängig von dem Konzentrationswert ermittelt werden. Die Substanz kann so lange gefördert werden, bis die Dosis gefördert ist. Die Substanz kann portionsweise gefördert werden. Dadurch kann eine Fördereinrichtung mit einer bestimmungsgemäßen Förderrate betrieben werden. Eine Fördereinrichtung mit fester Förderrate weist eine lange Standzeit und einen geringen Wartungsaufwand auf.

Die Substanz kann mit einer von dem Konzentrationswert abhängigen Förderrate gefördert werden. Die Förderrate kann umso höher sein, je größer ein Unterschied zwischen einem Sollwert der Konzentration und dem Konzentrationswert ist. Die Förderrate der Fördereinrichtung kann variabel sein. Beispielsweise kann eine Drehzahl eines Antriebsmotors der Fördereinrichtung variabel sein. Durch eine Regelung über den Unterschied wird eine Minimierung des Unterschieds erreicht. So kann die Konzentration der Substanz sehr nahe an einem Idealwert gehalten werden.

Das Verfahren kann einen Schritt des Erfassens des Konzentrationswerts aufweisen, in dem der Konzentrationswert basierend auf einer elektrischen Leitfähigkeit von Flüssigkeit in dem Kühlkreislauf erfasst wird. Die Substanz kann die elektrische Leitfähigkeit des Kühlwassers durch Freisetzen von Ionen beeinflussen. Beispielsweise kann die Leitfähigkeit mit zunehmender Konzentration der Substanz zunehmen. Ein Zusammenhang der Leitfähigkeit mit der Konzentration kann in einer Erfassungseinrichtung hinterlegt sein.

Der Konzentrationswert kann alternativ oder ergänzend basierend auf einer nachgefüllten Menge von Flüssigkeit in den Kühlkreislauf erfasst werden. Die nachgefüllte Flüssigkeit verdünnt die Substanz im Kühlwasser. Die nachzufördernde Menge der Substanz ist abhängig von der nachgefüllten Menge an Flüssigkeit. Durch die Erfassung der Flüssigkeitsmenge kann bereits Substanz gefördert werden, bevor eine Änderung der Konzentration erfasst werden kann.

Als Substanz kann Zitronensäure in den Kühlkreislauf gefördert werden. Zitronensäure kann bei einer Vielzahl von Materialien eingesetzt werden und weist ein hohes Lösungspotenzial für Kalk auf. Zitronensäure ist umweltfreundlich.

Weiterhin wird eine Vorrichtung zum Entkalken eines betriebsbereiten Kühlkreislaufs für eine betriebsbereite Vakuumpumpe für einen betriebsbereiten Sterilisator vorgestellt, wobei die Vorrichtung zumindest eine Einrichtung zum Ausführen des Verfahrens gemäß einem der vorhergehenden Ansprüche aufweist. Die Vorrichtung wird im Folgenden auch als Dosiervorrichtung zum Dosieren einer kalklösend wirkenden Substanz bezeichnet. Gemäß einer Ausführungsform umfasst die Vorrichtung eine Fördereinrichtung zum Fördern der Substanz aus einem Vorratsbehälter in einen Kühlkreislauf des Sterilisators und eine Steuereinrichtung zum Steuern der Fördereinrichtung.

Ferner wird ein Sterilisator vorgestellt, der eine Sterilisierkammer, eine mit der Sterilisierkammer verbundene Vakuumpumpe und einen mit der Vakuumpumpe verbundenen Kühlkreislauf aufweist, wobei eine Vorrichtung zum Entkalken des betriebsbereiten Kühlkreislaufs gemäß dem hier vorgestellten Ansatz mit dem Kühlkreislauf gekoppelt ist.

Die Förderung der kalklösend wirkenden Substanz in den Kühlkreislauf erfolgt vorzugsweise gesteuert von einer Steuereinrichtung des Sterilisators während des Betriebs der Vakuumpumpe mit einer mittels der Steuerung vorgegebenen oder vorgebbaren Dosis. Eine Förderung der kalklösend wirkenden Substanz kann insbesondere durch das Unterschreiten eines vorgegebenen Leitwertes der Flüssigkeit im Kühlkreislauf ausgelöst werden.

Ein Ausführungsbeispiel der Erfindung ist in den Zeichnungen rein schematisch dargestellt und wird nachfolgend näher beschrieben. Es zeigt
- Figur 1: eine Darstellung eines Sterilisators mit einer Vorrichtung zum Entkalken gemäß einem Ausführungsbeispiel; und
- Figur 2: ein Ablaufdiagramm eines Verfahrens zum Entkalken gemäß einem Ausführungsbeispiel.

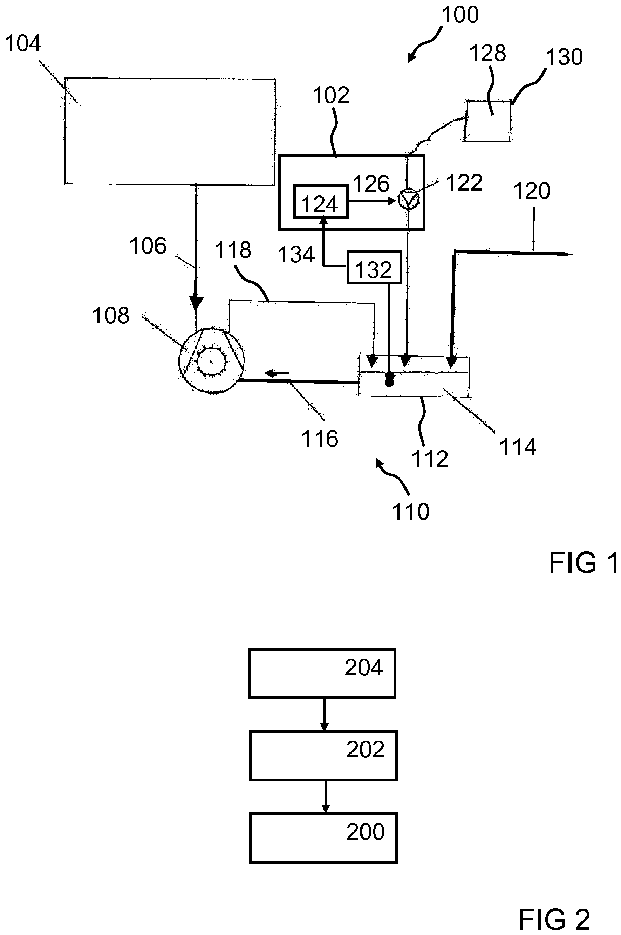

Figur 1 zeigt eine Darstellung eines Sterilisators 100 mit einer Vorrichtung 102 zum Entkalken gemäß einem Ausführungsbeispiel. Der Sterilisator 100 weist eine Sterilisierkammer 104 auf. Die Sterilisierkammer 104 ist über eine Vakuumleitung 106 mit einer Vakuumpumpe 108 verbunden. Die Vakuumpumpe 108 wird durch einen offenen Kühlkreislauf 110 gekühlt. Dazu wird der Vakuumpumpe 108 aus einem Vorlagebehälter 112 Betriebsflüssigkeit 114, beispielsweise Kühlwasser mit geringer Temperatur über eine Zuleitung 116 zugeleitet Über eine Rückleitung 118 wird die erwärmte Betriebsflüssigkeit 114 in den Vorlagebehälter 112 zurückgeführt. Verdampfende Betriebsflüssigkeit 114 wird über eine Kühlwasserzuleitung 120, beispielsweise aus der Wasserleitung ersetzt.

Die im Folgenden auch als Dosiervorrichtung 102 bezeichnete Vorrichtung 102 weist gemäß diesem Ausführungsbeispiel eine Fördereinrichtung 122 und eine Steuereinrichtung 124 auf. Die Steuereinrichtung 124 stellt ein Fördersignal 126 zum Steuern eines Betriebs der Fördereinrichtung 122 an eine Schnittstelle der Fördereinrichtung 122 bereit. Ansprechend auf einen Empfang des Fördersignals 126 ist die Fördereinrichtung 122 ausgebildet, um eine kalklösend wirkende Substanz 128 aus einem Vorratsbehälter 130 in den Kühlkreislauf 110 zu fördern. Die kalklösend wirkende Substanz 128 kann auch als Entkalkungsmittel bezeichnet werden.

Die Dosiervorrichtung 102 ist ausgebildet, um die Substanz 128 in den Vorlagebehälter 112 zu fördern. Ebenso kann die Dosiervorrichtung 102 mit dem Kühlwasserzulauf 120 oder der Zuleitung 116 verbunden sein und die Substanz 128 in den Kühlwasserzulauf 120 oder die Zuleitung 116 fördern.

Während des Förderns kann die Vakuumpumpe 108 in Betrieb oder in einem betriebsbereiten Zustand sein. Entsprechend kann der gesamte Sterilisator 100 während des Förderns in Betrieb oder in einem betriebsbereiten Zustand sein.

Gemäß einem Ausführungsbeispiel ist die Substanz 128 eine Flüssigkeit und die Fördereinrichtung 122 eine motorisch angetriebene Pumpe 122. Wenn der Vorratsbehälter 130 oberhalb des Vorlagebehälters 112 angeordnet ist, kann die Fördereinrichtung 122 auch ein über das Fördersignal 126 betätigbares Ventil sein. Gemäß einem weiteren Ausführungsbeispiel ist die Substanz 128 rieselfähig. In diesem Fall kann die Fördereinrichtung 122 eine motorisch angetriebene Förderschnecke und/oder Zellradschleuse sein.

Ist ein Verbrauch der Substanz 128 während eines Betriebs des Sterilisators 100 bekannt, kann die Fördereinrichtung 122 während des Betriebs von der Steuereinrichtung 124 nach einem festen Zeitplan angesteuert werden. Dabei kann die Fördereinrichtung 122 zu festgelegten Startzeitpunkten oder nach einer festgelegten Betriebsdauer des Sterilisators 100 über das Fördersignal 126 aktiviert werden, für eine festgelegte Zeitdauer die Substanz 128 in den Kühlkreislauf 110 fördern und wird anschließend wieder über das Fördersignal 126 deaktiviert werden, um die Zugabe der Substanz 128 zu stoppen. Alternativ dazu kann das Fördersignal 126 verwendet werden, um unter Verwendung der Fördereinrichtung 122 ein Volumenstrom beziehungsweise ein Mengenstrom der Substanz 128 einzustellen, der einem bekannten oder angenommenen Verlust der Substanz 128 aus dem Kühlkreislauf 110 entspricht.

Ist der Verbrauch der Substanz 128 während des Betriebs unbekannt, kann die Fördereinrichtung 122 in Abhängigkeit von einer Konzentration der Substanz 128 im Kühlkreislauf 110 angesteuert werden. Dazu weist der Sterilisator 100 eine Erfassungseinrichtung 132 auf, die die Konzentration erfasst und in einem Konzentrationswert 134 abbildet. Der Konzentrationswert 134 wird von der Steuereinrichtung 124 eingelesen und das Fördersignal 126 in Abhängigkeit von dem Konzentrationswert 134 bereitgestellt.

Die Erfassungseinrichtung 132 ist gemäß einem Ausführungsbeispiel ausgebildet, um einen elektrischen Leitwert des Kühlwassers 114 zu messen und den auf Basis des elektrischen Leitwerts bestimmten Konzentrationswert 134 auszugeben. Alternativ oder ergänzend ist die Erfassungseinrichtung 132 ausgebildet, um eine Menge des nachfließenden Leitungswassers aus der Kühlwasserzuleitung 120 zu erfassen und den Konzentrationswert 134 über eine resultierende Verdünnung des Kühlwassers 114 im Vorlagebehälter 112 zu bestimmen.

In einem Ausführungsbeispiel wird dabei unter Verwendung des Konzentrationswerts 134 in der Steuereinrichtung 124 eine notwendige Dosis der Substanz 128 bestimmt, um die Konzentration auf einen Sollwert anzuheben. Die Fördereinrichtung 122 wird über das Fördersignal 126 so lange aktiviert, bis die Dosis in den Kühlkreislauf 110 eingebracht ist.

In einem Ausführungsbeispiel wird in der Steuereinrichtung 124 unter Verwendung des Konzentrationswerts 134 eine momentane Fördermenge der Fördereinrichtung 122 über das Fördersignal 126 umso größer eingestellt, je größer eine Differenz zwischen dem Sollwert und der tatsächlichen Konzentration ist.

Im Folgenden wird eine Entkalkung der Vakuumpumpe 108 anhand eines Ausführungsbeispiels des Sterilisators in Form eines Dampf-Großsterilisators 100 beschrieben. Ohne Entkalken kann die Vakuumpumpe 108 des Dampf-Großsterilisators 100 verkalken und einen vorzeitigen Leistungsverlust aufweisen.

Die Vakuumpumpe 108 des Dampf-Großsterilisators 100 wird direkt mit Leitungswasser oder mit einem Gemisch aus Leitungswasser und Kondensat gekühlt. Beispielsweise kann dabei ein Härtegrat des Wassers zwischen 5°dH und 15°dH angestrebt werden. Je nach Wasserqualität kann es schon bei 10°dH bis 12°dH zu einer Bildung von Kalkbelag auf den Laufrädern und den Steuerscheiben der Pumpe 108 und damit zum vorzeitigen Leistungsverlust kommen. Um das Verkalken zu vermindern, kann der Sterilisator 100 an eine zentrale Wasseraufbereitung beispielsweise eines Krankenhauses angeschlossen werden.

Ohne den hier vorgestellten Ansatz kann ein regelmäßiger Ausbau der Pumpe 108 und ein Spülen und Entkalken mit einer kalklösenden Flüssigkeit erforderlich sein. Der Dampf-Großsterilisator 100 ist in dieser Zeit stillgelegt.

Durch den hier vorgestellten Ansatz wird das Verkalken der Vakuumpumpe 108 unabhängig von der Qualität des Kühlwassers 114 verhindert und somit die Standzeit erhöht. Ein Ausbau der Pumpe 108 zum Entkalken ist nicht erforderlich.

Das Verkalken der Vakuumpumpe 108 und damit vorzeitiger Leistungsverlust wird verhindert, indem dem Kühlkreislauf 110 eine bestimmte Menge einer oder mehrerer kalklösender Substanzen 128, wie beispielsweise Zitronensäure diskontinuierlich oder kontinuierlich mittels einer Dosiereinrichtung 122 zugeführt wird. Damit wird die Kalkablagerung sicher verhindert. Die Konzentration der kalklösenden Substanz 128 kann beispielsweise permanent mittels Messung des Leitwerts des Kühlwassers 114 reguliert werden. Ebenso kann die erforderliche Dosierung über den Verbrauch an Kühlwasser 114, der entsprechend gemessen wird, berechnet werden.

Die Steuerung der Dosierung kann mit einer unabhängigen, eigenen Steuerung 124 der Dosiereinrichtung 122 oder direkt aus einer Steuerung des Sterilisators 100 erfolgen.

Die Dosierung kann in den Umlaufbehälter 112, die Leitung 116 für die Betriebsflüssigkeit oder die Kühlwasserleitung 120 erfolgen.

Ein vorzeitiger Leistungsverlust der Pumpe 108 durch Kalkablagerungen wird verhindert. Eine vorgeschaltete Wasseraufbereitung ist nicht mehr notwendig. Es ergeben sich reduzierte Wartungskosten und Ersatzteilkosten durch eine erhöhte Lebensdauer der Pumpe 108.

Figur 2 zeigt ein Ablaufdiagramm eines Verfahrens zum Entkalken gemäß einem Ausführungsbeispiel. Das Verfahren kann unter Verwendung einer mit einem Kühlkreislauf eines Sterilisators verbundenen Vorrichtung 102, wie sie in Figur 1 dargestellt ist, ausgeführt werden. Das Verfahren weist einen Schritt 200 des Förderns auf. Dabei wird zumindest eine kalklösend wirkende Substanz aus einem Vorratsbehälter in den Kühlkreislauf der Pumpe des Sterilisators dosiert.

In einem Ausführungsbeispiel weist das Verfahren einen Schritt 202 des Ermittelns auf, in dem eine zu dosierende Dosis der Substanz abhängig von einem Konzentrationswert ermittelt wird. Dabei wird im Schritt 200 des Förderns die Substanz so lange gefördert, bis die Dosis gefördert ist.

In einem Ausführungsbeispiel weist das Verfahren einen Schritt 204 des Erfassens auf. Dabei wird der Konzentrationswert basierend auf einer elektrischen Leitfähigkeit von Flüssigkeit in dem Kühlkreislauf und/oder basierend auf einer nachgefüllten Menge von Flüssigkeit in den Kühlkreislauf erfasst.

## Patentansprüche

1. Verfahren zum Entkalken eines betriebsbereiten Kühlkreislaufs (110), mit dem eine Vakuumpumpe (108) eines Sterilisators (100), gekühlt wird, wobei das Verfahren den folgenden Schritt umfasst:
Fördern (200) mittels einer motorisch angetriebenen Pumpe (122) zumindest einer kalklösend wirkenden Substanz (128) aus einem Vorratsbehälter (130) in den Kühlkreislauf (110),
wobei während des Förderns die Vakuumpumpe (108) und der Sterilisator (100) in Betrieb sind.

2. Verfahren gemäß Anspruch 1, bei dem im Schritt (200) des Förderns die Substanz (128) unter Verwendung eines vorgegebenen Zeitplans gefördert wird.

3. Verfahren gemäß Anspruch 1, bei dem im Schritt (200) des Förderns die Substanz (128) abhängig von einem, eine Konzentration der Substanz (128) in dem Kühlkreislauf (110) repräsentierenden Konzentrationswert (134) gefördert wird.

4. Verfahren gemäß Anspruch 3, mit einem Schritt (202) des Ermittelns einer zu dosierenden Dosis der Substanz (128), wobei die Dosis abhängig von dem Konzentrationswert (134) ermittelt wird und im Schritt (200) des Förderns die Substanz (128) so lange gefördert wird, bis die Dosis gefördert ist.

5. Verfahren gemäß Anspruch 3, bei dem im Schritt des Förderns (200) die Substanz (128) mit einer von dem Konzentrationswert (134) abhängigen Förderrate gefördert wird, wobei die Förderrate umso höher ist, je größer ein Unterschied zwischen einem Sollwert der Konzentration und dem Konzentrationswert (134) ist.

6. Verfahren gemäß einem der Ansprüche 3 bis 5, mit einem Schritt (204) des Erfassens des Konzentrationswerts (134), wobei der Konzentrationswert (134) basierend auf einer elektrischen Leitfähigkeit von Flüssigkeit (114) in dem Kühlkreislauf (110) erfasst wird.

7. Verfahren gemäß einem der Ansprüche 3 bis 6, mit einem Schritt (204) des Erfassens des Konzentrationswerts (134), wobei der Konzentrationswert (134) basierend auf einer nachgefüllten Menge von Flüssigkeit in den Kühlkreislauf (110) erfasst wird.

8. Verfahren gemäß einem der vorhergehenden Ansprüche, bei dem im Schritt (200) des Förderns als Substanz (128) Zitronensäure in den Kühlkreislauf (110) gefördert wird.

9. Sterilisator (100), der eine Sterilisierkammer (104), eine mit der Sterilisierkammer (104) verbundene Vakuumpumpe (108) und einen mit der Vakuumpumpe (108) verbundenen Kühlkreislauf (110) aufweist, wobei eine Vorrichtung (102) zum Entkalken des Kühlkreislaufs (110) in Betrieb mit dem Kühlkreislauf (110) gekoppelt ist, die zumindest eine Steuerung ausgebildet (122, 124) zum Ausführen des Verfahrens gemäß einem der vorhergehenden Ansprüche aufweist.

## Claims

1. Method for decalcifying an operational cooling circuit (110) by means of which a vacuum pump (108) of a steriliser (100) is cooled, wherein the method comprises the following step:
conveying (200) at least one lime-dissolving substance (128) from a storage container (130) into the cooling circuit (110) by means of a motor-driven pump (122), wherein during the conveying the vacuum pump (108) and the steriliser (100) are in operation.

2. Method according to claim 1, in which, in the conveying step (200), the substance (128) is conveyed using a predetermined schedule.

3. Method according to claim 1, in which, in the conveying step (200), the substance (128) is conveyed on the basis of a concentration value (134) which represents a concentration of the substance (128) in the cooling circuit (110).

4. Method according to claim 3, comprising a step (202) of determining a dose of the substance (128) to be dosed, wherein the dose is determined on the basis of the concentration value (134), and in the conveying step (200) the substance (128) is conveyed until the dose has been conveyed.

5. Method according to claim 3, in which, in the conveying step (200), the substance (128) is conveyed at a conveying rate which is dependent on the concentration value (134), wherein the greater a difference between a desired value of the concentration and the concentration value (134), the higher the conveying rate.

6. Method according to any of claims 3 to 5, comprising a step (204) of detecting the concentration value (134), wherein the concentration value (134) is detected based on an electrical conductivity of liquid (114) in the cooling circuit (110).

7. Method according to any of claims 3 to 6, comprising a step (204) of detecting the concentration value (134), wherein the concentration value (134) is detected based on a replenished amount of liquid in the cooling circuit (110).

8. Method according to any of the preceding claims, in which, in the conveying step (200), citric acid is conveyed into the cooling circuit (110) as the substance (128).

9. Steriliser (100) comprising a sterilisation chamber (104), a vacuum pump (108) connected to the sterilisation chamber (104), and a cooling circuit (110) connected to the vacuum pump (108), wherein a device (102) for decalcifying the cooling circuit (110) is coupled to the cooling circuit (110) in operation and has at least one controller (122, 124) for carrying out the method according to any of the preceding claims.

## Revendications

1. Procédé de détartrage d'un circuit de refroidissement opérationnel (110) permettant de refroidir une pompe à vide (108) d'un stérilisateur (100), le procédé comprenant l'étape consistant à :
transporter (200) dans le circuit de refroidissement (110), au moyen d'une pompe entraînée de façon motorisée (122), au moins une substance (128) ayant un effet de dissolution de calcaire, à partir d'un réservoir (130), la pompe à vide (108) et le stérilisateur (100) étant en fonctionnement pendant le transport.

2. Procédé selon la revendication 1 dans lequel, à l'étape (200) de transport, la substance (128) est transportée selon un programme prédéterminé.

3. Procédé selon la revendication 1, dans lequel, à l'étape (200) de transport, la substance (128) est transportée en fonction d'une valeur de concentration (134) représentant une concentration de la substance (128) dans le circuit de refroidissement (110).

4. Procédé selon la revendication 3, comprenant une étape (202) de détermination d'une dose de la substance (128) à doser, la dose étant déterminée en fonction de la valeur de concentration (134), et la substance (128) étant transportée aussi longtemps à l'étape (200) de transport, jusqu'au transport de la dose.

5. Procédé selon la revendication 3, dans lequel, à l'étape (200) de transport, la substance (128) est transportée à un débit de transport dépendant de la valeur de concentration (134), plus le débit de transport étant élevé, plus la différence entre une valeur de consigne de la concentration et la valeur de concentration (134) étant grande.

6. Procédé selon l'une des revendications 3 à 5, comprenant une étape (204) de détection de la valeur de concentration (134), la valeur de concentration (134) étant détectée sur la base d'une conductivité électrique du liquide (114) dans le circuit de refroidissement (110).

7. Procédé selon l'une des revendications 3 à 6, comprenant une étape (204) de détection de la valeur de concentration (134), la valeur de concentration (134) étant détectée sur la base d'une quantité ajoutée de liquide dans le circuit de refroidissement (110).

8. Procédé selon l'une des revendications précédentes, dans lequel, à l'étape (200) de transport, de l'acide citrique est transporté en tant que substance (128) dans le circuit de refroidissement (110).

9. Stérilisateur (100) comprenant une chambre de stérilisation (104), une pompe à vide (108) connectée à la chambre de stérilisation (104) et un circuit de refroidissement (110) connecté à la pompe à vide (108), dans lequel un dispositif (102) de détartrage du circuit de refroidissement (110) est couplé en fonctionnement avec le circuit de refroidissement (110), et comprend au moins un dispositif de commande (122, 124) pour la mise en oeuvre du procédé selon l'une des revendications précédentes.
